Europäisches Patentamt

European Patent Office

Office européen des brevets

(19)

(11) **EP 1 338 287 A1**

(12) **EUROPEAN PATENT APPLICATION**
published in accordance with Art. 158(3) EPC

(43) Date of publication:
**27.08.2003 Bulletin 2003/35**

(51) Int Cl.⁷: **A61K 38/02**, A61K 9/50,
A61K 9/10, A61K 47/30,
C07K 1/00

(21) Application number: **01976660.9**

(22) Date of filing: **11.10.2001**

(86) International application number:
**PCT/JP01/08911**

(87) International publication number:
**WO 02/030449 (18.04.2002 Gazette 2002/16)**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **12.10.2000 JP 2000312167**

(71) Applicant: **TANABE SEIYAKU CO., LTD.
Chuo-ku, Osaka-shi, Osaka 541-8505 (JP)**

(72) Inventors:
• **MATSUMOTO, Akihiro
Hirakata-shi, Osaka 537-0064 (JP)**
• **MORITA, Takahiro
Izumi-shi, Osaka 594-0074 (JP)**

(74) Representative: **HOFFMANN - EITLE
Patent- und Rechtsanwälte
Arabellastrasse 4
81925 München (DE)**

(54) **METHOD OF FORMING POLYPEPTIDE PARTICLES**

(57)     The present invention is to provide polypeptide fine particles which comprise adding a polypeptide-insoluble water-miscible organic solvent to a frozen product of an aqueous solution containing a polypeptide and a phase separation inducer, and dissolving the separation inducer and ice in the frozen product; a polypeptide fine particle dispersion obtainable by the method; polypeptide fine particles obtainable by removing the phase separation inducer, water and water-miscible organic solvent from the polypeptide fine particle dispersion; a method of producing microspheres which comprises dispersing the polypeptide fine particles in a solution in which a biodegradable polymer is dissolved in a volatile and water-miscible organic solvent, emulsifying the mixture in an aqueous phase to prepare an O/W emulsion, and removing the water-miscible organic solvent; and a microsphere preparation obtainable by the method.

Fig. 2

EP 1 338 287 A1

## Description

Technical field

The present invention relates to a method of making fine particles of polypeptide.

Background art

In recent years, polypeptides having physiological activity have come to be widely used in treatments and preventions of various diseases of humans and animals.

Since these polypeptides have short half-lives in the body, and are hard to be absorbed from the digestive tract following an oral administration and so forth, they have conventionally been administered by multiple injections. However, simple and convenient administration methods have been studied to overcome the problems such as complexity of the administration method, burden on the patient.

Transpulmonary administration is one example of such methods. However, in the case of transpulmonary administration, it is necessary to form the polypeptide into fine particles in order to allow the polypeptide to reach inside the pulmonary alveoli.

In addition, studies have also been actively conducted on the delivery of the polypeptide into the body over a long period of time by sealing the polypeptide in a biodegradable polymer, forming microspheres and then implanting them in the body by injection.

Although a method is known for producing such microspheres, which consists of emulsifying an aqueous solution of a polypeptide in an organic solvent such as methylene chloride in which is dissolved a biodegradable polymer to prepare a W/O emulsion, and emulsifying this in water to obtain microspheres, this method has the problem of the polypeptide losing its activity as a result of denaturation, thereby allowing the method to be applied to the limited kinds of the polypeptides.

In order to solve this problem, the method of preparation of microspheres has been examined consisting of dispersing a polypeptide in the form of a powder directly in an organic solvent in which a biodegradable polymer has been dissolved to obtain an oil phase, and using this oil phase to prepare microspheres. In this method, however, if the particle size of the polypeptide is excessively large, it ends up with the microspheres being unable to achieve a high encapsulation efficiency, as well as releasing a large amount of the polypeptide during the early stage of dissolution (initial burst phenomenon), thereby making it necessary to form the polypeptide into fine particles in the same manner as an aerosol preparation.

The following methods are known so far as the methods for forming a polypeptide into fine particles.

(I) A method comprising forming fine particles by spray drying an aqueous solution containing a water-soluble polymer substance such as gelatin and polypeptide (Japanese Patent Laid-Open Publication No. 4-36233).
(II) A method comprising freeze-drying an aqueous solution containing a polypeptide and a water-soluble polymer substance, and pulverizing the resulting freeze-dried product with a jet mill (Japanese Patent Laid-Open Publication No. 8-225454).
(III) A method comprising adding an aqueous polypeptide solution into acetone and crystallizing the polypeptide fine particles (Journal of Microencapsulation, Vol. 14, No. 2, pp. 225-241, 1997).
(IV) A method comprising mixing a surfactant and a polypeptide followed by rapid-drying (Japanese Patent Laid-Open Publication No. 9-315997).
(V) A method comprising adding a water-miscible organic solvent or volatile salt to an aqueous polypeptide solution followed by freeze-drying (Japanese Patent Laid-Open Publication No. 11-322631).
(VI) A method comprising freeze-drying a mixed aqueous solution of a polypeptide and polyethylene glycol followed by dissolving polyethylene glycol with an organic solvent (Japanese Patent Laid-Open Publication No. 11-302156).

In all of these methods, however, there are problems including loss of an activity of the polypeptide, low yield, poor re-dispersibility or a considerable amount of time being required for preparation, and a method that resolves all of these problems has still not been found out.

An object of the present invention is to provide a method for producing polypeptide fine particles that is simple, fast, has a high yield and is not accompanied by denaturation.

Summary of the invention

When an aqueous solution containing a polypeptide and a phase separation inducer is frozen, phase separation occurs between both components during the course of the freezing, and a frozen product is obtained in a state in which micro-droplets of the polypeptide aqueous solution are dispersed in an aqueous solution of the phase separation inducer. The inventors of the present invention found out that, when a polypeptide-insoluble, water-miscible organic solvent is added to this frozen product, the phase separation inducer and ice dissolve in this organic solvent, and finally, the polypeptide fine particles are obtained in the form of dispersion.

According to the method of the present invention, in addition to being able to obtain the polypeptide fine particles easily, rapidly and at a high yield, the resulting fine particles retain a high level of activity and also have excellent re-dispersibility.

Namely, the present invention relates to a method of making a polypeptide fine particle dispersion which comprises adding a polypeptide-insoluble, water-miscible organic solvent to a frozen product of an aqueous solution containing a polypeptide and a phase separation inducer, and dissolving the phase separation inducer and ice in said frozen product.

Also, the present invention relates to polypeptide fine particles obtainable by removing the phase separation inducer, water and water-miscible organic solvent from the polypeptide fine particle dispersion obtainable by the above-mentioned method.

The present invention further relates to a method of producing microspheres which comprises dispersing the above-mentioned polypeptide fine particles in a solution in which a biodegradable polymer is dissolved in a volatile, water-immiscible organic solvent, dispersing this in an aqueous phase to prepare an O/W emulsion, and removing said water-immiscible organic solvent.

The present invention also relates to a method of producing microspheres which comprises adding a polymer solution containing the above-mentioned polypeptide fine particles, a biodegradable polymer and a good solvent for said polymer which solvent is miscible with water (Solvent A), to a homogeneously mixed liquid containing a poor solvent for said polymer which solvent is miscible with said Solvent A (Solvent B) and a poor solvent for said polymer which solvent is immiscible with said Solvent A (Solvent C), emulsifying said mixture to prepare an emulsion, in which a polymer solution forms a dispersed phase and a homogeneously mixed liquid forms a continuous phase, and removing the solvent A from said dispersed phase.

The present invention further relates to a microsphere preparation obtainable by the above-mentioned method.

Brief description of the drawings

Fig. 1 is a graph showing the relationship between a concentration ratio (PEG6000/BSA) of a BSA-PEG6000 aqueous solution and an obtained BSA fine particle size.
Fig. 2 is a schematic drawing of an electron micrograph of BSA fine particles obtained according to the method of the present invention (Example 1).
Fig. 3 is a graph showing the size distribution of BSA fine particles in a BSA fine particle dispersion obtained according to the method of the present invention, and in a dispersion in which BSA fine particles obtained from the above dispersion as a powder were re-dispersed.
Fig. 4 is a graph showing the results of a dissolution test of microspheres (Example 15) prepared using the BSA fine particles obtained according to the method of the present invention.
Fig. 5 is a schematic drawing of an electron micrograph of the BSA fine particles obtained according to a reference method for comparison (Comparative Example 1).

Best mode for carrying out the invention

In the present invention, an aqueous solution (to be referred to as a polypeptide-phase separation inducer aqueous solution) is first prepared that contains a polypeptide and a phase separation inducer.

Examples of polypeptides include various peptides or proteins having a physiological activity that is useful for mammals therefore, being able to be used clinically. Polypeptides having a molecular weight as a monomer of, for example, 1,000-200,000, and particularly preferably 5,000-70,000, are generally used for said polypeptide. These polypeptides also include polymers classified as a protein which is said to have a higher order structure in the field of biochemistry. Although there are no particular restrictions on the types of polypeptides used in the present invention provided the object of the present invention is achieved, those having a some solubility in water are preferable, and that solubility in water is about 0.01 mg/ml or more, and preferably about 1 mg/ml or more, at 20°C.

Specific examples of polypeptides include hormones, enzymes, cytokines and growth factors. More specifically, these include the polypeptides indicated below.

Examples of hormones may include growth hormone (GH), growth hormone releasing factor (GRF), insulin, glucagon, gastrin, prolactin, adrenocorticotropic hormone (ACTH), thyroid stimulating hormone (TSH), follicle-stimulating hormone (FSH), leuteinizing hormone (LH), human chorionic gonadotropin (hCG) and calcitonin.

Examples of enzymes may include tissue plasminogen activator (tPA), urokinase (UK), streptokinase, protein C, metalloproteases, super oxide dismutase (SOD), factor VIII, factor IX and peroxidase.

Examples of cytokines may include interferons (such as IFN-$\alpha$ -$\beta$ and -$\gamma$), interleukins (such as IL-1 through IL-11), cachectin, oncostatin, colony stimulating factors (such as G-, M- and GM-CSF), thrombopoietin (TPO) and erythropoietin (EPO).

Examples of growth factors may include neural growth factors (such as NGF-1 and NGF-2), neurotropic factor (NTF), epithelial growth factor (EGF), platelet-derived growth factor (PDGF), insulin-like growth factors (such as IGF-1, IGF-2 and IGF-3), fibroblast growth factors (aFGF and bFGF), osteogen growth factors (such as BMP-1, BMP-2, BMP-3 and BMP-4), atrial natriuretic peptide (ANP) and cartilage inducing factor.

These polypeptides may have sugar chains and those sugar chain structures may be different.

Moreover, these include muteins, derivatives, analogues and active fragments, etc.

The polypeptides used in the present invention may either be of natural origin or obtained by gene recombination technology.

In addition, the polypeptides used in the present invention may be metal salts, and examples of metals in said metal salts may include alkaline earth metals (such as calcium and magnesium), zinc (divalent), iron (divalent or trivalent), copper (divalent), tin (divalent or tetravalent) and aluminum (divalent or trivalent), etc. and polypeptides in the form of metal salts are included in polypeptide in the present specification.

There are no particular restrictions on the phase separation inducer used in the present invention provided that, when a polypeptide and a phase separation inducer are dissolved in water at high concentrations (for example, 200 mg/ml or more of a polypeptide and 50 mg/ml or more of a phase separation inducer), it induces phase separation between the polypeptide-rich phase and phase separation inducer-rich phase. An example of such a phase separation inducer is non-peptide, water-soluble polymer.

Those non-peptide, water-soluble polymers having a mean molecular weight of 400-200,000, and preferably 6,000-70,000, are used preferably, and specific examples of such non-peptide, water-soluble polymers include polyoxyethylenes, cellulose derivatives, polyvinyl derivatives and cyclodextrin derivatives.

Examples of polyoxyethylenes may include polyethylene glycol, polyoxyethylene hydrogenated castor oil, polyoxyethylene-polyoxypropylene glycol, and polyoxyethylene sorbitan fatty acid ester, etc.

Examples of cellulose derivatives may include hydroxypropyl cellulose, etc.

Examples of polyvinyl derivatives may include polyvinyl pyrrolidone and polyvinyl alcohol, etc.

Examples of cyclodextrin derivatives may include dimethyl-$\beta$-cyclodextrin, etc.

Particularly preferable examples of phase separation inducers include polyethylene glycol and polyvinyl pyrrolidone, and the most preferable example is polyethylene glycol. Polyethylene glycol preferably has a mean molecular weight within the range of 400-200,000, and more preferably within the range of 6,000-70,000.

Not only one type of phase separation inducer, but also a combination of two or more types of phase separation inducers may be used.

Aqueous solution of a polypeptide and a phase separation inducer may be prepared either by dissolving both polypeptide and phase separation inducer in water all at once, by dissolving one followed by dissolving the other, or by mixing a polypeptide aqueous solution and a phase separation inducer aqueous solution. What is essential is that, ultimately, the polypeptide and the phase separation inducer be dissolved in water, and there are no particular restrictions on the preparation method.

There are no particular restrictions on the concentration of the polypeptide in the polypeptide-phase separation inducer aqueous solution as long as it is within a range that allows the polypeptide to be dissolved. If a specific indication is required, the concentration of the polypeptide would be 0.01-200 mg/ml, and preferably 1-100 mg/ml.

There are no particular restrictions on the concentration of the phase separation inducer in the polypeptide-phase separation inducer aqueous solution. If a specific indication is required, the concentration of the phase separation inducer would be 0.025-200 mg/ml, and preferably 0.25-100 mg/ml.

Next, the polypeptide-phase separation inducer aqueous solution is frozen. Then, a polypeptide-insoluble, water-miscible organic solvent is added to the resulting frozen product. Finally, the phase separation inducer and ice are dissolved to obtain the target polypeptide fine particles in a form of a dispersion.

In the freezing of the polypeptide-phase separation inducer aqueous solution, it is preferable to employ a condition under which the temperature of the aqueous solution is lowered gradually rather than suddenly. These conditions can be easily achieved by freezing the solution using a normally used freezer or freeze-dryer at a set temperature of -20 to -80°C. To indicate these conditions more specifically, the initial rate of the lowering temperature (temperature of the aqueous solution lowered per minute after the start of cooling) is about 2-40°C/min, and preferably about 10-30°C/min.

In the freezing process of the method of the present invention, since solidification initially begins from the water in the polypeptide-phase separation inducer aqueous solution, concentration of the polypeptide and the phase separation inducer elevates in the non-ice portion. As a result, phase separation occurs to form a polypeptide-rich phase and a phase separation inducer-rich phase.

This phase separation phenomenon consists of the case in which the polypeptide-rich phase forms micro-droplets in the phase separation inducer-rich phase, or the case in which the phase separation inducer-rich phase forms micro-droplets in the polypeptide-rich phase. Which form of the phase separation occurs is determined according to the concentration ratio of phase separation inducer/polypeptide in the polypeptide-phase separation inducer aqueous solution (to be referred to as the concentration ratio). If this concentration ratio is greater than or equal to some value that is determined according to the types of the polypeptides and the phase separation inducers (to be referred to as the phase transition ratio), the polypeptide-rich phase forms micro-droplets in the phase separation inducer-rich phase.

In general, if the concentration ratio is equal to or greater than the phase transition ratio, spherical fine particles are obtained having a smaller particle size. On the other hand, in the case the concentration ratio is equal to or less than the phase transition ratio, although particles are obtained, their shape is irregular and their particle size is somewhat large. Thus, it is preferable that the concentration ratio be equal to or greater than the phase transition ratio in order to obtain fine particles having a smaller particles size, such as a mean particle size of 10 μm or less.

Although the phase transition ratio varies according to the types of the polypeptides and the phase separation inducer, it can be easily determined by suitably preparing fine particles using the polypeptide-phase separation inducer aqueous solutions of various concentration ratios, and examining their particle sizes and shapes.

In the case of using polyethylene glycol having a mean molecular weight of 6,000 or more for the phase separation inducer, the phase transition ratio can be estimated to a certain extent according to the molecular weight of the polypeptide. Namely, the following relational equation is approximately valid between the molecular weight of the polypeptide (M) and the phase transition ratio (T):

$$T = 10^{2.7}/M^{0.68}$$

In this equation, the phase transition ratio can be determined by entering the molecular weight of the polypeptide used.

As described above, although the phase transition ratio varies according to the types of the polypeptides and the phase separation inducers and so forth, the concentration ratio in order to obtain fine particles having a mean particle size of 10 μm or less, would be 0.25 of more, preferably 0.5 or more, and more preferably 1 or more.

In the present invention, since the polypeptide phase is dispersed in the phase separation inducer phase at the stage when the polypeptide-phase separation inducer aqueous solution is frozen, polypeptide is obtained in the form of fine particles by only dissolving the ice and the phase separation inducer present in the frozen product. A polypeptide-insoluble, water-miscible organic solvent is added to said frozen product to dissolve the ice and the phase separation inducer in said frozen product. Although the case of (a) in which the ice dissolves directly in the organic solvent, and the case of (b) in which the ice mixes into the organic solvent after melting to water, can occur for the ice in the frozen product as a result of addition of said organic solvent, the dissolving of ice in the present invention is not limited to the cases of (a) only, but rather includes the case of (b) as well provided the water is immediately mixed into the organic solvent.

There are no particular restrictions on the polypeptide-insoluble, water-miscible organic solvent provided that it does not dissolve the polypeptide, but dissolves the ice and the phase separation inducer when added to a frozen product of the polypeptide-phase separation inducer aqueous solution. This organic solvent should, for example, be completely miscible with water, have polypeptide solubility of 1 mg/ml or less at 20°C, and should not cause precipitation of the phase separation inducer even when five times of the volume of said organic solvent are added to a 100 mg/ml phase separation inducer aqueous solution. In addition, this organic solvent should be suitably selected according to the types of the polypeptides and the phase separation inducers. Specific examples of such organic solvents include lower alcohols such as methanol, ethanol, 1-propanol, 2-propanol and 2-methyl-2,4-pentanediol, ketones such as acetone, ethers such as tetrahydrofuran and dioxane, acetonitrile, pyridine, dimethylformamide, and dimethylsulfoxide. Of these, acetone, acetonitrile, methanol, ethanol and 2-methyl-2,4-petanediol are preferable, while acetone is particularly preferable. These organic solvents may also be used as a mixture of two or more kinds.

It is necessary that the amount of the water-miscible organic solvent added should be sufficient, so that the moisture content of the water-containing organic solvent, obtained by dissolving the ice in the frozen product in water-miscible organic solvent, remains at a level at which polypeptide is unable to re-dissolve. Although this amount depends on the types of the polypeptides, the phase separation inducers and the water-miscible organic solvents and so forth, the amount should normally be one time to 100 times, and preferably 2-20 times of the amount of the water in the polypeptide-phase separation inducer aqueous solution.

In the present invention, although the phase separation inducer and the ice are dissolved by adding a water-miscible organic solvent, this can be done rapidly by stirring after an addition of an organic solvent. In addition, organic solvent may also be added while stirring. Stirring can be performed using existing stirring means such as a magnetic stirrer, propeller, touch mixer or ultrasonic waves.

Replacement of dispersion medium or removal of the phase separation inducer and water in the dispersion can also be performed for the resulting polypeptide fine particle dispersion using a known means, such as dialysis, centrifugal separation and washing, filtration and washing, and so on, if required. In addition, the fine particles can also be obtained as a powder by removing the dispersion medium (the water-miscible organic solvent containing water and the phase separation inducer) from the dispersion, using a means such as filtration and centrifugal separation, followed by drying.

The resulting polypeptide fine particles can be used in the form of a spray preparation, microsphere preparation and so forth.

Spray preparations are prepared only with polypeptide fine particles removed from the dispersion as powder, or by adding a surfactant to improve dispersibility of the fine particles. Examples of the surfactants may include sorbitan trioleate, soybean lecithin, oleoyl alcohol and hydrogenated castor oil derivatives. The amount blended is within the range of 0.001-5% (w/w), and preferably within the range of 0.05-2% (w/w), relative to the weight of the polypeptide.

In addition, stabilizer, excipient and so forth may be added as necessary. Examples of stabilizers may include human serum albumin and gelatin, and the amount blended is preferably 0.01-200% (w/w) of the weight of the polypeptide. Examples of excipients include sugars and sugaralcohols such as lactose, maltose, sorbitose, trehalose, xylose, mannitol, sorbitol and xylitol, and the amount blended is within the range of 0.01-200% (w/w), and preferably within the range of 1-50% (w/w), relative to the weight of the polypeptide.

Moreover, mineral ions required to maintain an osmotic pressure inherently present in the body may be suitably blended, examples of whicyh include calcium ion, magnesium ion, sodium ion, potassium ion and chloride ion. In addition, a surfactant of alveolar origin may also be blended as necessary, in order to suppress irritation to the body.

A polypeptide composition for a spray preparation prepared in this manner is inhaled into the pharyngeal region or the pulmonary region via the oral or nasal cavity either as it is, or by using compressed air or compressed carbon dioxide gas as a spray gas, or by using a suitable propellant to disperse the composition. Examples of the propellants that can be used may include chlorofluorocarbons (Flon 11, 12, 114, etc.), hydrogen-containing chlorofluorocarbons (Flon 123, 124, 141b, etc.), and chlorine-free fluorocarbons (Flon 125, 134a, etc.).

To use the composition in the form of a microsphere preparation, it can be prepared using the methods described below.

(A) Spray Drying Method

An organic solvent solution of a biodegradable polymer in which polypeptide fine particles are dispersed is sprayed into the drying chamber of a spray dryer using a spray nozzle to evaporate the organic solvent in the sprayed droplets in an extremely short period of time (Japanese Patent Laid-Open Publication No. 1-155942, Japanese Patent Laid-Open Publication No. 8-151321, Japanese Patent Laid-Open Publication No. 9-221417, Drug Development and Industrial Pharmacy, Vol. 24(8), pp. 703-727, 1998 (to simply be referred to as Reference 1 in the following), etc.).

(B) Phase Separation Method (Coacervation Method)

A coacervation agent is gradually added to an organic solvent solution of a biodegradable polymer in which polypeptide fine particles are dispersed while stirring to precipitate and solidify microspheres (Japanese Patent Laid-Open Publication No. 60-67417, Japanese Patent Laid-Open Publication No. 8 151321, Japanese Patent Laid Open Publication No. 9-221417, the above Reference 1, etc.).

(C) In Liquid Drying Method

Polypeptide fine particles are dispersed in a solution in which a biodegradable polymer is dissolved in a volatile, water-immiscible organic solvent. Then, this is dispersed in an aqueous phase to prepare an O/W emulsion, followed by evaporation of said organic solvent (Japanese Patent Laid-Open Publication No. 49-12024, Japanese Patent Laid-Open Publication No. 63-91325, Japanese Patent Laid-Open Publication No. 8-151321, the above Reference 1, etc.). In the other method, two or more types of biodegradable polymers are dissolved in the same or different volatile, water-immiscible organic solvent(s), and after dispersing polypeptide fine particles in one solution, both are mixed to prepare an O/O emulsion. This O/O emulsion is then dispersed in an aqueous phase to prepare an O/O/W emulsion followed by evaporation of said organic solvent (Japanese Patent Laid-Open Publication No. 6-211648).

(D) Microsphere Preparation Method Using Water-Miscible Organic Solvent

A polymer solution, containing polypeptide fine particles, a biodegradable polymer and a good solvent of the above polymer that is miscible with water (Solvent A), is added to a uniformly mixed liquid containing a poor solvent of the above polymer that is miscible with Solvent A (Solvent B) and a poor solvent of the above polymer that is not miscible with-Solvent A (Solvent C) followed by emulsifying to prepare an emulsion in which the polymer solution forms a dispersed phase and the uniformly mixed liquid forms a continuous phase, thereby allowing microspheres to be prepared by removing Solvent A from the dispersed phase (Japanese Patent Application No. 2000-122469).

In the preparation of microspheres according to the method of (D), a polymer solution is first prepared that contains polypeptide fine particles, a biodegradable polymer and a good solvent of the above polymer that is miscible with water (Solvent A).

The amount of polypeptide fine particles in the polymer solution is 0.001-90 wt%, and preferably 0.01-50 wt%.

Any biodegradable polymers that are conventionally used in the pharmaceutical field can be used for the biodegradable polymer in the present invention. Preferable examples of such include polylactic acid and lactic acid-glycolic acid copolymer. The concentration of biodegradable polymer in the polymer solution is normally 1-80 wt%, and preferably 20-60 wt%.

There are no particular restrictions on the good solvent for said biodegradable polymer which solvent is miscible with water (Solvent A) provided that the amount of the solvent A required to completely dissolve 1 g of biodegradable polymer is less than 25 g and that it has the property of being completely miscible with water. Preferable examples of such include acetone and tetrahydrofuran, with acetone being the most preferable. Only one type of these solvents or a mixture of two or more types of these solvents may be used.

Next, by adding the prepared polymer solution to a homogeneously mixed liquid containing a poor solvent for said biodegradable polymer which solvent is miscible with Solvent A (Solvent B) and a poor solvent for said biodegradable polymer which solvent is not miscible with Solvent A (Solvent C) and emulsifying, an emulsion is prepared in which the polymer solution forms a dispersed phase and the homogeneously mixed liquid forms a continuous phase.

There are no particular restrictions on Solvent B provided that the amount of the solvent B required to completely dissolve 1 g of biodegradable polymer is 25 g or more and that it is completely miscible with Solvent A. Specific examples of such are water and ethanol, with water being particularly preferable.

In addition, there are no particular restrictions on Solvent C provided that the amount of the solvent C required to completely dissolve 1 g of biodegradable polymer is 25 g or more, that the amount of Solvent A that is miscible with 100 parts by weight of Solvent C is 25 parts by weight or less, and that it is completely miscible with Solvent B to form a homogeneously mixed liquid. A specific example of which is glycerin.

The weight ratio of Solvent B and Solvent C in the homogeneously mixed liquid is preferably within the range of 10:90 to 50:50, and most preferably within the range of 20:80 to 40:60.

In addition, said homogeneously mixed liquid may also contain an emulsification stabilizer, preferable examples of which include polyvinyl alcohol, polyvinyl pyrrolidone, methyl cellulose and hydroxypropyl cellulose.

Although Solvent A is partially miscible with the homogeneously mixed liquid of Solvent B and Solvent C, since the rate of mixing is restricted, an emulsion can be formed in which the polymer solution containing drug, biodegradable polymer and Solvent A forms a dispersed phase, and the homogeneously mixed liquid forms a continuous phase. Simultaneous to emulsion formation, or in parallel with emulsion formation, Solvent A is gradually removed from the polymer solution into the homogeneously mixed liquid, and microsphere formation begins.

The weight ratio of the polymer solution and the homogeneously mixed liquid is preferably within the range of 1:2 to 1:200, and particularly preferably within the range of 1:3 to 1:75.

Emulsification of the polymer solution into the homogeneously mixed liquid can be easily performed by adding the polymer solution to the homogeneously mixed liquid while stirring by a known emulsification apparatus such as a propeller type stirrer, turbine emulsifier, high pressure emulsifier, ultrasonic dispersing apparatus or static mixer. Although the amount of time required for emulsification varies according to the emulsification apparatus used, amount of liquid and so forth, it is about 1-10 minutes. Emulsification can also be suitably carried out by methods such as membrane emulsification and spraying.

Following emulsification, the resulting emulsion is fluidized by an appropriate method, and the remaining Solvent A is removed from the dispersed phase. As a result, Solvent A of the dispersed phase is removed into the continuous phase, and the biodegradable polymer of the dispersed phase completely solidifies to obtain microspheres.

Circulation or stirring can be performed for the fluidization method. Circulation can be carried out by suctioning a portion of the emulsion from the bottom of the emulsion using a pump, passing it through a pipe, and then returning it to the upper portion of the emulsion. Stirring can be carried out by an ordinary stirring means such as a stirring blade or magnetic stirrer.

In addition, following emulsification, the removal of Solvent A from the dispersed phase can be accelerated if the amount of the continuous phase of the already formed emulsion is increased. On top of that, by increasing the amount of the continuous phase, Solvent A in the continuous phase can be prevented from having a detrimental effect on the resulting microspheres, since Solvent A removed into the continuous phase during emulsification is diluted.

Moreover, the removal of Solvent A can be further accelerated if the temperature is increased, reduced pressure is applied and so forth when removing residual Solvent A from the dispersed phase of the emulsion after emulsification.

The resulting microspheres should be separated by such methods as centrifugal separation or filtration, washed with distilled water and air dried or vacuum dried, etc. until they are completely free of solvent.

The microspheres obtained by any one of the above-described methods (A) through (D) can be implanted parenterally as they are. In addition, they can also be used as raw materials during the production of various preparations. Examples of such preparations include injections, peroral preparations, percutaneous preparations, suppositories, pernasal preparations, oral cavity preparations and intraocular preparations.

Examples

The following provides a more detailed explanation of the present invention through its examples.

Example 1

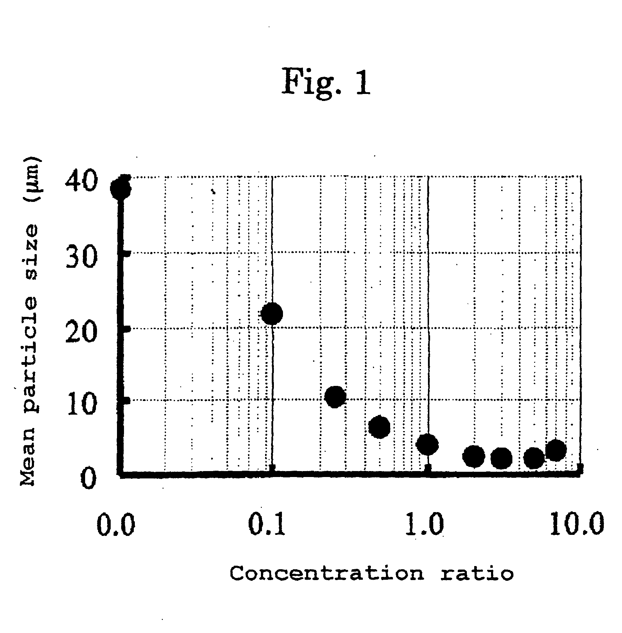

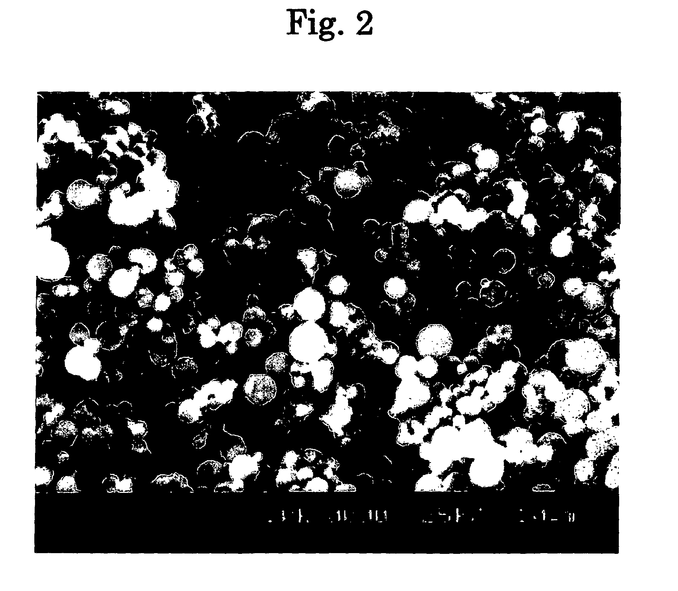

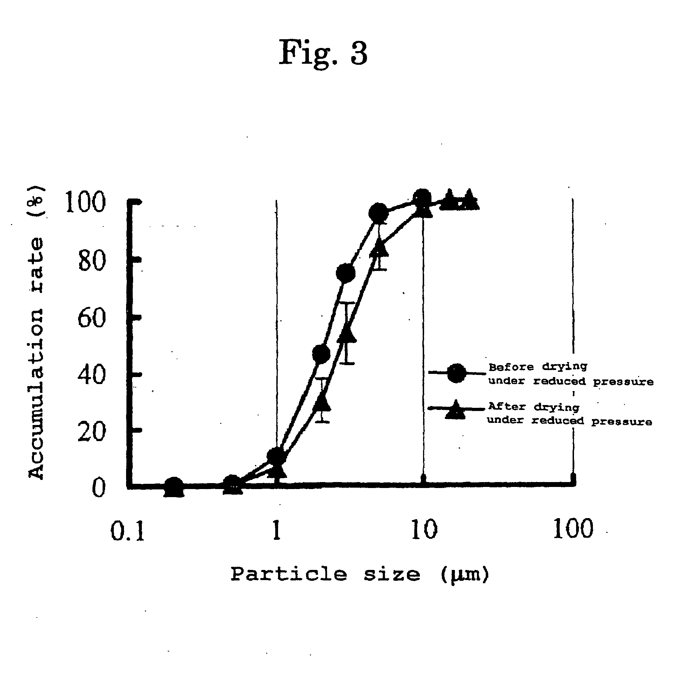

(1) One ml aliquots of a BSA-PEG6000 aqueous solution (PEG6000/BSA concentration ratio: 0-7) having a BSA (bovine serum albumin, molecular weight: 67,000, Sigma) concentration of 10 mg/ml and a PEG6000 (Polyethylene glycol 6000, mean molecular weight: 7,500, Wako Pure Chemical Industries) concentration of 0-70 mg/ml, were respectively prepared.
(2) After freezing these solutions at -20°C, 5 ml of acetone pre-chilled at -20°C were added followed by stirring at room temperature using a touch mixer to dissolve the PEG6000 and ice finally to obtain a BSA fine particle dispersion. After separating the dispersion by centrifugation (2,000 rpm, 5 minutes), and removing the supernatant, 2 ml of acetone were added to obtain a BSA fine particle dispersion (after removal of PEG6000 and water).
(3) The mean particle size of the fine particles in the resulting dispersion (after removal of PEG6000 and water) obtained in (2) was measured using a laser diffraction particle size distribution measuring system (SALD-1100, Shimadzu). Those results are shown in Fig. 1. Fine particles having a mean particle size of about 10 μm or less were obtained at the concentration ratios of the BSA-PEG6000 aqueous solutions (PEG6000/BSA) of 0.25 and above.
(4) After separating the BSA fine particle dispersion obtained in (2) (after removal of PEG6000 and water) by centrifugation (2,000 rpm, 5 minutes) and removing the supernatant, the BSA fine particles were dried under reduced pressure overnight, and the BSA fine particles were recovered in the form of a dry powder.
(5) A schematic drawing of an electron micrograph of the BSA fine particles (concentration ratio: 3) obtained in (4) is shown in Fig. 2. It can be understood from Fig. 2 that the BSA fine particles obtained according to the present invention have a spherical shape.
(6) When the BSA fine particles (concentration ratio: 3) obtained in (4) were dispersed in acetone, they promptly re-dispersed. The mean particle size of the re-dispersed BSA fine particles was 2.85 μm, which was nearly the same as that prior to drying under reduced pressure, namely, the same as the size of the fine particles in the fine particle dispersion obtained in (2) (2.09 μm). In addition, there was also hardly any change in particle size distribution before and after drying under reduced pressure (Fig. 3).

Example 2

One ml of a BSA-PEG1000 aqueous solution (concentration ratio: 3) was prepared in which the BSA concentration was 10 mg/ml and the PEG1000 (Polyethylene glycol 1000, molecular weight: 950-1,050, Nippon Yushi) concentration was 30 mg/ml, followed by performing the same procedure as in step (2) of Example 1 to obtain a BSA fine particle dispersion (after removal of PEG1000 and water) having a mean particle size of 4.50 μm.

Example 3

One ml of a BSA-PEG2000 aqueous solution (concentration ratio: 3) was prepared in which the BSA concentration was 10 mg/ml and the PEG2000 (Polyethylene glycol 2000, molecular weight: 1,800-2,200, Katayama Chemical) concentration was 30 mg/ml, followed by performing the same procedure as in step (2) of Example 1 to obtain a BSA fine particle dispersion (after removal of PEG2000 and water) having a mean particle size of 4.06 μm.

Example 4

One ml of a BSA-PEG4000 aqueous solution (concentration ratio: 3) was prepared in which the BSA concentration was 10 mg/ml and the PEG4000 (Polyethylene glycol 4000, molecular weight: 2,700-3,400, Katayama Chemical) concentration was 30 mg/ml, followed by performing the same procedure as in step (2) of Example 1 to obtain a BSA fine particle dispersion (after removal of PEG4000 and water) having a mean particle size of 2.58 μm.

Example 5

One ml of a BSA-PEG20000 aqueous solution (concentration ratio: 3) was prepared in which the BSA concentration was 10 mg/ml and the PEG20000 (Polyethylene glycol 20000, molecular weight: 19,000, Katayama Chemical) concentration was 30 mg/ml, followed by performing the same procedure as in step (2) of Example 1 to obtain a BSA fine particle dispersion (after removal of PEG20000 and water) having a mean particle size of 2.33 μm.

Example 6

One ml of a BSA-PEG70000 aqueous solution (concentration ratio: 3) was prepared in which the BSA concentration was 10 mg/ml and the PEG70000 (Polyethylene glycol 70000, molecular weight: 70,000, Wako Pure Chemical Industries) concentration was 30 mg/ml, followed by performing the same procedure as in step (2) of Example 1 to obtain a BSA fine particle dispersion (after removal of PEG70000 and water) having a mean particle size of 2.30 μm.

Example 7

One ml of a BSA-PEG6000 aqueous solution (concentration ratio: 3) was prepared in which the BSA concentration was 10 mg/ml and the PEG6000 concentration was 30 mg/ml, followed by performing the same procedure as in step (2) of Example 1 with the exception of using methanol in place of acetone to obtain a BSA fine particle dispersion (after removal of PEG6000 and water) having a mean particle size of 2.96 μm.

Example 8

One ml of a BSA-PEG6000 aqueous solution (concentration ratio: 3) was prepared in which the BSA concentration was 10 mg/ml and the PEG6000 concentration was 30 mg/ml, followed by performing the same procedure as in step (2) of Example 1 with the exception of using ethanol in place of acetone to obtain a BSA fine particle dispersion (after removal of PEG6000 and water) having a mean particle size of 2.72 μm.

Example 9

One ml of an aqueous solution (concentration ratio: 3) was prepared having a BSA concentration of 10 mg/ml and a Pluronic F68 [Polyoxyethylene (mean degree of polymerization: approx. 160)-polyoxypropylene (mean degree of polymerization: approx. 30) copolymer, molecular weight: 5,900-12,500, Asahi Denka Kougyo] concentration of 30 mg/ml, followed by performing the same procedure as in step (2) of Example 1 to obtain a BSA fine particle dispersion (after removal of Pluronic F68 and water) having a mean particle size of 4.15 μm.

Example 10

One ml of an aqueous solution (concentration ratio: 3) was prepared having a BSA concentration of 10 mg/ml and a HPC-SL (Hydroxypropyl cellulose, Shin-Etsu Chemical) concentration of 30 mg/ml, followed by performing the same procedure as in step (2) of Example 1 to obtain a BSA fine particle dispersion (after removal of HPC-SL and water) having a mean particle size of 3.69 μm.

Example 11

One ml of an aqueous solution (concentration ratio: 3) was prepared having a BSA concentration of 10 mg/ml and a Kollidon K30 (polyvinyl pyrrolidone, mean molecular weight: 40,000, BASF) concentration of 30 mg/ml, followed by performing the same procedure as in step (2) of Example 1 to obtain a BSA fine particle dispersion (after removal of Kollidon K30 and water) having a mean particle size of 2.72 μm.

Example 12

One ml of an aqueous solution (concentration ratio: 3) was prepared having a BSA concentration of 10 mg/ml and a dimethyl-β-cyclodextrin (Nacalai tesque) concentration of 30 mg/ml, followed by performing the same procedure as in step (2) of Example 1 to obtain a BSA fine particle dispersion (after removal of dimethyl-β-cyclodextrin and water) having a mean particle size of 9.24 μm.

Example 13

One ml of an aqueous solution (concentration ratio: 3) was prepared having a BSA concentration of 10 mg/ml and a Tween 20 (polyoxyethylene sorbitan fatty acid ester, Wako Pure Chemical Industries) concentration of 30 mg/ml, followed by performing the same procedure as in step (2) of Example 1 to obtain a BSA fine particle dispersion (after removal of Tween 20 and water) having a mean particle size of 4.77 μm.

Example 14

One ml of an aqueous solution (concentration ratio: 10) was prepared having an HRP (horseradish peroxidase, molecular weight: approx. 40,000, Wako Pure Chemical Industries) concentration of 10 mg/ml and a PEG6000 concentration of 100 mg/ml, followed by performing the same procedure as in step (2) of Example 1 to obtain an HRP fine particle dispersion (after removal of PEG6000 and water) having a mean particle size of 2.42 μm. After separating by centrifugation (2,000 rpm, 5 minutes) and removing the supernatant, the HRP fine particles were dried under reduced pressure for 3 hours at room temperature and the HRP fine particles were recovered in the form of a dry powder.
When the activity of the HRP fine particles was measured using the Sumilon "Peroxidase Coloring Kit O" (Sumitomo Bakelite), they were determined to retain nearly 100% of their activity.

Example 15

(1) 200 mg of BSA was dissolved in 14 ml of water, and it was added and mixed in 6 ml of an aqueous solution of 10% (w/w) PEG6000. The resulting mixture was frozen at -20°C. 100 ml of acetone prechilled at -20°C were added followed by stirring at room temperature using a magnetic stirrer to dissolve the PEG6000 and water (ice) and in the acetone to obtain a BSA fine particle dispersion. After separating by centrifugation (2,000 rpm, 5 minutes) and removing the supernatant, 40 ml of acetone were added followed by centrifugal separation (2,000 rpm, 5 minutes), removal of the supernatant and drying under reduced pressure overnight to obtain 195.1 mg of BSA fine particles having a mean particle size of 4.15 $\mu$m in the form of a dry powder.

(2) After dispersing 25 mg of the resulting BSA fine particles in 1350 mg of methylene chloride, 475 mg of PLGA5020 (lactic acid-glycolic acid copolymer, mean molecular weight: 20,000, lactic acid/glycolic acid molar ratio: 50/50, Wako Pure Chemical Industries) were added and dissolved. This was then added to 4 ml of an aqueous solution of 0.25% (w/w) methyl cellulose (Metholose SM-4000, Shin-Etsu Chemical) at 15°C, and emulsified using a Polytron homogenizer (Kinematica AG Littau, 8,000 rpm, 3 minutes). The resulting emulsion was added to 400 ml of water at 15°C, and dried in liquid by raising the temperature to 30°C over the course of 3 hours while stirring with a propeller stirrer (Three One Motor BL600, Heidon, 400 rpm) to let microspheres to form. The resulting microsphere dispersion went under filtration using a filter having a mesh size of 150 $\mu$m, and the microspheres were collected with a filter having a mesh size of 20 $\mu$m followed by freeze-drying.

(3) When the encapsulation efficacy of BSA in the resulting microspheres was measured with a micro BCA protein assay kit (PIERCE), 84.9% of the loaded amount of BSA was found to be contained in the microspheres.

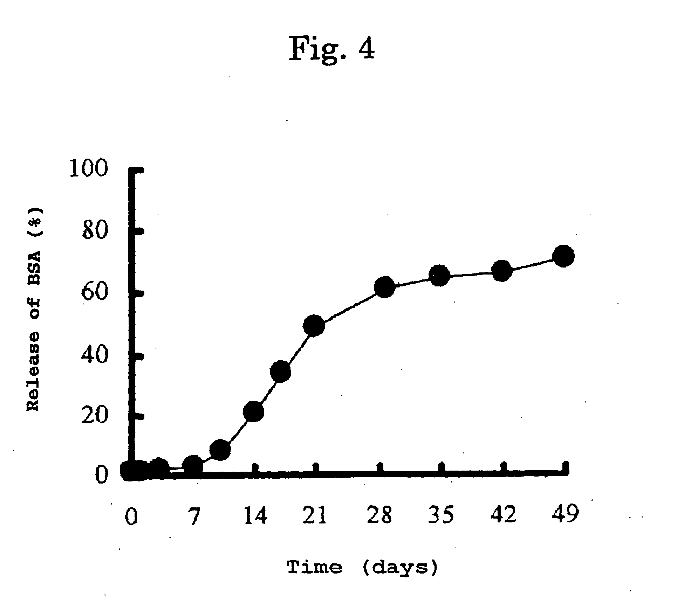

(4) 10 mg of the microspheres were placed in a test tube with cap seal followed by the addition of 10 ml of PBS (9.6 mM phosphate-buffered saline). A dissolution test was then conducted using a rotary incubator (Taitech, 25 rpm, 37°C). The test tube was periodically removed from the rotary incubator and separated by centrifugation (2,000 rpm, 5 minutes) after which a 9 ml sample of the supernatant was obtained. After sampling, 9 ml of fresh PBS were added and the dissolution test was continued. The amount of BSA in the sampled supernatant was measured with a Micro BCA Protein Assay Kit to determine the amount of BSA released from the microspheres. Those results are shown in Fig. 4.

Example 16

After dispersing 25 mg of the BSA fine particles obtained in (1) of Example 15 in 800 mg of acetone, 475 mg of PLGA5020 (lactic acid-glycolic acid copolymer, mean molecular weight: 20,000, lactic acid/glycolic acid molar ratio: 50/50, Wako Pure Chemical Industries) were added and dissolved to prepare a polymer solution. This was added to 4 g of a glycerin-water mixture (weight ratio of glycerin and water: 70:30) containing polyvinyl alcohol (1.0 wt%), and emulsified using a propeller stirrer (Three One Motor BL600, Heidon, 1,500 rpm, 3 minutes) to obtain an emulsion in which the polymer solution formed the dispersed phase and the glycerin-water mixture formed the continuous phase. This emulsion was added to 14 g of glycerin-water mixture (weight ratio of glycerin and water: 70:30), stirred for 2.5 hours with a magnetic stirrer, followed by an addition of 10 ml of water, stirring for 0.5 hours and removal of the acetone from the dispersed phase of the emulsion to obtain a dispersion of microspheres. After filtering this dispersion with a 150 $\mu$m filter and collecting the microspheres with a 20 $\mu$m filter, freeze-drying was performed to obtain microspheres.

When the encapsulation efficacy of BSA in the resulting microspheres was measured by the same method as in part (3) of Example 15, 79.1% of the loaded amount of BSA was contained in the microspheres.

Comparative Example

(1) 1 ml of an aqueous solution having a BSA concentration of 10 mg/ml and a PEG6000 concentration of 30 mg/ml (concentration ratio: 3) was prepared. 5 ml of acetone pre-chilled at -20°C was added followed by stirring at room temperature using a touch mixer to obtain a BSA fine particle dispersion. After separating by centrifugation (2,000 rpm, 5 minutes) and removing the supernatant, 2 ml of acetone were added to obtain a BSA fine particle dispersion (after removal of PEG6000 and water). The mean particle size of the resulting fine particles was 2.58 $\mu$m.

(2) After separating the BSA fine particle dispersion (after removal of PEG6000 and water) obtained in (1) by centrifugation (2,000 rpm, 5 minutes) and removing the supernatant, the resulting fine particles were dried under reduced pressure overnight to obtain BSA fine particles in the form of a dry powder.

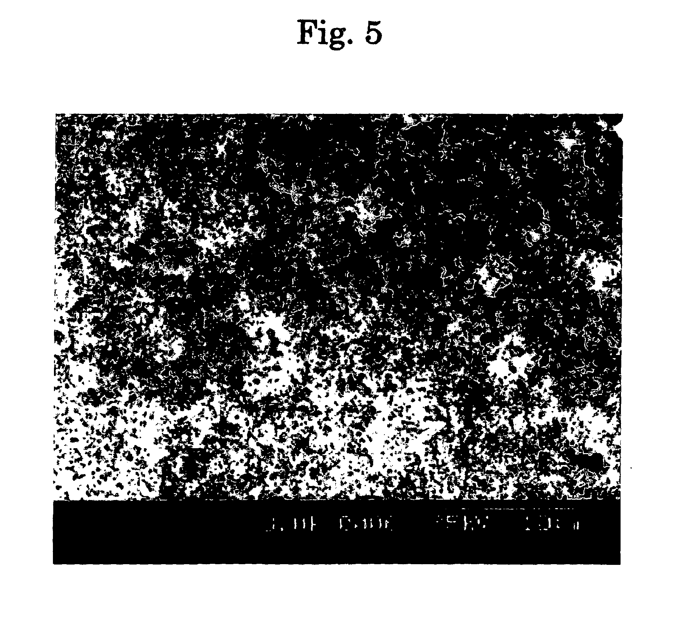

(3) A schematic drawing of an electron micrograph of the resulting BSA fine particles is shown in Fig. 5. It can be understood from Fig. 5 that spherical fine particles are not obtained unless freezing is performed.

Moreover, although the resulting BSA fine particles were attempted to be dispersed in acetone, they were not dispersed at all.

Utilizability in industry

According to the present invention, polypeptide fine particles can be obtained easily, rapidly and without any loss of activity, and the resulting polypeptide fine particles are of extremely high purity and have satisfactory re-dispersibility.

In addition, since the method of the present invention results in hardly any loss during recovery of the fine particles, the recovery rate is nearly 100%.

Moreover, use of the polypeptide fine particles of the present invention enables high-content microspheres to be obtained easily, which have an ability to release the polypeptide at a fixed rate over a long period of time, with an extremely low initial burst rate.

## Claims

1. A method of producing a polypeptide fine particle dispersion which comprises adding a polypeptide-insoluble water-miscible organic solvent to a frozen product of an aqueous solution containing a polypeptide and a phase separation inducer, and dissolving the phase separation inducer and ice in said frozen product.

2. The method according to Claim 1, wherein said phase separation inducer is a non-peptide water-soluble polymer.

3. The method according to Claim 2, wherein said non-peptide water-soluble polymer is one kind or two or more kinds selected from polyoxyethylenes, cellulose derivatives, polyvinyl derivatives and cyclodextrin derivatives.

4. The method according to Claim 3, wherein said polyoxyethylene is polyethylene glycol, polyoxyethylene hydrogenated castor oil, polyoxyethylene-polyoxypropylene copolymer or polyoxyethylene sorbitan fatty acid ester, said cellulose derivative is hydroxypropylcellulose, said polyvinyl derivative is polyvinyl pyrrolidone or polyvinyl alcohol, and said cyclodextrin derivative is dimethyl-β-cyclodextrin.

5. The method according to Claim 1, wherein said phase separation inducer is polyethylene glycol.

6. The method according to Claim 5, wherein an average molecular weight of said polyethylene glycol is 400 to 200,000.

7. The method according to Claim 5, wherein an average molecular weight of said polyethylene glycol is 6,000 to 70,000.

8. The method according to any one of Claims 1 to 7, wherein said polypeptide-insoluble water-miscible organic solvent is one kind or two or more kinds selected from the group consisting of methanol, ethanol, 1-propanol, 2-propanol, 2-methyl-2,4-pentanediol, acetone, tetrahydrofuran, dioxane, acetonitrile, pyridine, dimethylformamide and dimethylsulfoxide.

9. The method according to any one of Claims 1 to 8, wherein a concentration ratio of phase separation inducer/ polypeptide in the aqueous solution containing polypeptide and phase separation inducer is equal to or greater than the phase inversion ratio.

10. The method according to any one of Claims 1 to 8, wherein a concentration ratio of phase separation inducer/ polypeptide in the aqueous solution containing a polypeptide and a phase separation inducer is 0.25 or more.

11. The method according to Claim 7, wherein a concentration ratio of phase separation inducer/ polypeptide in the aqueous solution containing a polypeptide and a phase separation inducer is $10^{2.7}/M^{0.68}$
    wherein M represents a molecular weight of polypeptide,
    or more.

12. A polypeptide fine particle dispersion obtainable by the method according to any one of Claims 1 to 11.

13. The polypeptide fine particle dispersion according to Claim 12, wherein said polypeptide fine particles are spherical fine particles having an average particle size of 10 μm or less.

14. Polypeptide fine particles obtainable by removing the phase separation inducer, water and water-miscible organic solvent from the polypeptide fine particle dispersion obtainable by the method according to any one of Claims 1 to 11.

15. A method of producing microspheres which comprises dispersing the polypeptide fine particles according to Claim 14 in a solution in which a biodegradable polymer is dissolved in a volatile, water-immiscible organic solvent, dispersing this in an aqueous phase to prepare an O/W emulsion, and removing said water-immiscible organic solvent.

16. A method of producing microspheres which comprises adding a polymer solution containing the polypeptide fine particles according to Claim 14, a biodegradable polymer and a good solvent for said polymer which solvent is miscible with water (Solvent A), to a homogeneously mixed liquid containing a poor solvent B for said polymer which solvent is miscible with said Solvent A and a poor solvent C for said polymer which solvent is immiscible with said Solvent A, emulsifying said mixture to prepare an emulsion, in which a polymer solution forms a dispersed phase and a homogeneously mixed liquid forms a continuous phase, and removing the solvent A from said dispersed phase.

17. A microsphere preparation obtainable by the method according to Claim 15 or 16.

# Fig. 1

Fig. 2

# Fig. 3

Fig. 4

Fig. 5

## INTERNATIONAL SEARCH REPORT

| International application No. |
| --- |
| PCT/JP01/08911 |

**A. CLASSIFICATION OF SUBJECT MATTER**
Int.Cl⁷ A61K37/02, A61K9/50, A61K9/10, A61K47/30, C07K1/00

According to International Patent Classification (IPC) or to both national classification and IPC

**B. FIELDS SEARCHED**

Minimum documentation searched (classification system followed by classification symbols)
Int.Cl⁷ A61K37/02, A61K9/50, A61K9/10, A61K47/30, C07K1/00

Documentation searched other than minimum documentation to the extent that such documents are included in the fields searched
Jitsuyo Shinan Koho          1922-1996     Toroku Jitsuyo Shinan Koho   1994-2001
Kokai Jitsuyo Shinan Koho   1971-2001     Jitsuyo Shinan Toroku Koho   1996-2001

Electronic data base consulted during the international search (name of data base and, where practicable, search terms used)
CA(STN),EMBASE(STN),MEDLINE(STN),BIOSIS(STN)

**C. DOCUMENTS CONSIDERED TO BE RELEVANT**

| Category* | Citation of document, with indication, where appropriate, of the relevant passages | Relevant to claim No. |
| --- | --- | --- |
| X | JP 11-302156 A (Tanabe Seiyaku Co., Ltd.), 02 November, 1999 (02.11.99), | 1-14 |
| Y | Full text   (Family: none) | 15-17 |
| Y | JP 11-171787 A (Teikoku Hormone Mfg. Co., Ltd.), 29 June, 1999 (29.06.99), Full text   (Family: none) | 15,17 |
| Y | EP 582459 B (Takeda Chemical Industries, Ltd.), 07 January, 1998 (07.01.98), Full text & JP 6-145046 A     & US 5611971 A & CA 203518 A     & DE 69316101 A | 16-17 |

☐ Further documents are listed in the continuation of Box C.     ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 12 December, 2001 (12.12.01) | 25 December, 2001 (25.12.01) |

| Name and mailing address of the ISA/ | Authorized officer |
| --- | --- |
| Japanese Patent Office | |
| Facsimile No. | Telephone No. |

Form PCT/ISA/210 (second sheet) (July 1992)